# EUROPÄISCHE PATENTANMELDUNG

(11) **EP 4 817 572 A1**
(43) Veröffentlichungstag der Anmeldung: **07.10.2026**
(21) Anmeldenummer: 26162880.4
(22) Anmeldetag: 06.03.2026
(51) Int. Cl.: A61G 1/04, A61G 1/044, A61G 7/07

(54) **VORRICHTUNG ZUR FIXIERUNG EINES LEBEWESENS**

(30) Priorität: 04.04.2025 AT 502412025
(71) Anmelder: Kohlbrat & Bunz Gesellschaft m.b.H, 5550 Radstadt (AT)
(72) Erfinder: GRAF, Michael, 5584 Zederhaus (AT)
(74) Vertreter: Torggler & Hofmann Patentanwälte - Innsbruck

(57) **Zusammenfassung**

Vorrichtung (1) zur Fixierung eines Lebewesens, insbesondere eines Neugeborenen, umfassend
- ein Grundelement (2) mit wenigstens einem Befestigungsmittel (3) zur Befestigung der Vorrichtung (1) an einer Transportvorrichtung (4), vorzugsweise einer Trage oder einem Sitz, und
- wenigstens ein am Grundelement (2) angeordnetes, flexibles Anlageelemente (5), wobei das Anlageelement (5) am Lebewesen festlegbar ist,
wobei das Grundelement (2) wenigstens einen ersten evakuierbaren oder aufblasbaren Innenraum (6) und das wenigstens eine Anlageelement (5) wenigstens einen zweiten evakuierbaren Innenraum (7) aufweist, wobei der wenigstens eine erste Innenraum (6) und der wenigstens eine zweite Innenraum (7) gesondert voneinander ausgebildet und/oder gesondert voneinander evakuierbar sind.

## Beschreibung

Die vorliegende Erfindung betrifft eine Vorrichtung zur Fixierung eines Lebewesens, insbesondere eines Neugeborenen, umfassend
- ein Grundelement mit wenigstens einem Befestigungsmittel zur Befestigung der Vorrichtung an einer Transportvorrichtung, vorzugsweise einer Trage oder einem Sitz, und
- wenigstens ein am Grundelement angeordnetes, flexibles Anlageelemente, wobei das Anlageelement am Lebewesen festlegbar ist,
wobei das Grundelemente wenigstens einen ersten evakuierbaren oder aufblasbaren Innenraum und das wenigstens eine Anlageelement wenigstens einen zweiten evakuierbaren Innenraum aufweist. Zudem betrifft die Erfindung auch ein Verfahren zum Fixieren eines Lebewesens mit einer solchen Vorrichtung.

Notfalltransporte von Neugeborenen oder Frühgeborenen in Rettungstransportfahrzeugen stellen für Rettungsorganisationen eine sehr große und schwierige Aufgabe dar. Das ist insbesondere aufgrund der hohen Anforderungen im Bereich des Transportes hinsichtlich Erschütterungen, Temperaturerhalt, Möglichkeit zur Beatmung und Bestehen eines 10G-Crash-Test der Fall.

Beim einem 10G-Crash-Test wird überprüft, ob ein Lebewesen, in diesem Fall ein Neugeborenes, einen Aufprall mit der zehnfachen Erdbeschleunigung (10G) übersteht, wenn es sich in einer entsprechenden Vorrichtung befindet. Ist dies der Fall, genügt die Vorrichtung den Erfordernissen eines 10G-Crash-Tests.

Insbesondere diese letztgenannte sicherheitstechnische Betrachtungsweise findet bei gattungsgemäßen Vorrichtungen immer größere Bedeutung. Demgemäß besteht die Notwendigkeit eines Rückhaltesystems für die Sicherung von Neugeborenen in Fahrzeugen, welche sowohl die medizinischen Anforderungen als auch die Anforderungen hinsichtlich der genannten sicherheitstechnischen Aspekte erfüllt.

Die ideale Ausführung einer solchen Vorrichtung dahingehenden sind spezielle Inkubator für Neugeborene, welche für den Einsatz in Fahrzeugen geeignet sind. In der Praxis sind dazu speziell ausgerüstete Rettungsfahrzeuge (Kinder-Rettungswagen) vorgesehen. Diese sind jedoch nicht in ausreichender Anzahl und somit nicht flächendeckend für Rettungsorganisationen verfügbar. Zudem können solche Fahrzeuge ausschließlich für den Transport von Früh-Neugeborenen bzw. Säuglingen verwendet werden und dienen meist für den Transfer zwischen Kliniken. Mobile Inkubatoren, wie sie in Hubschraubern zum Einsatz kommen, dienen auch nur zum Transport zwischen Kliniken.

Für den üblichen und planmäßigen Rettungsdienst werden aktuelle andere Systeme in Form von verschieden ausgebildeten Krankentragen für den Notfalltransport verwendet, um (erwachsene) Patienten im Rettungsfahrzeug zur Notaufnahme zu transportieren.

Es sind auch Systeme bekannt, um Neugeborene mittels Gurtsystemen und Polsterungen an bestehende Krankentragen im Fahrzeug zu fixieren. Bei diesen Systemen handelt es sich meist um reine Gurtlösungen, um zumindest eine gewisse Rückhaltung während des Transportes zu ermöglichen. Es sind auch Rückhaltesysteme bekannt, welche mit speziellen Vakuumatratzen kombinierbar sind.

Eine Fixierung von Neugeborenen in solchen Systemen erfüllt, selbst mit eingelegter spezieller Vakuumatratze, speziell angepassten Gurten und Schnalle oder unter Verwendung von Vakuumatratzen mit eingebauten Fixiermöglichkeit, nicht die medizinischen und sicherheitstechnischen Anforderungen.

Insbesondere besteht die Problematik darin, dass Neugeborene hinsichtlich ihrer der Körpergröße zu klein sind, um mit Gurt- oder Schnallensystemen sicher fixiert zu werden. Es ist nahezu unmöglich, die Gurte zu fixieren, ohne dass eine Schnalle oder sonstiges am Patienten anlegt, und eben zu Druckstelle oder im schlimmsten Fall Verletzungen führen kann.

Aufgabe der Erfindung ist es daher, eine gattungsgemäße Vorrichtung anzugeben, welche in herkömmlichen (Rettungs-)Fahrzeugen zum Einsatz kommen kann und die neuen medizinischen und sicherheitstechnischen Anforderungen erfüllt. Darüber hinaus soll auch ein Verfahren zum Fixieren eines Lebewesens mit einer solchen Vorrichtung angegeben werden.

Diese Aufgaben werden gelöst durch die Merkmale der Ansprüche 1 und 14.

Erfindungsgemäß ist bei einer wie oben beschriebenen Vorrichtung vorgesehen, dass der wenigstens eine erste Innenraum und der wenigstens eine zweite Innenraum gesondert voneinander ausgebildet und/oder gesondert voneinander evakuierbar sind.

Das Grundelement und das Anlageelement können also gesondert voneinander evakuiert und auch geformt werden. Das Grundelement kann somit als Grundlage zur Fixierung des Lebewesens als auch zur Befestigung der Vorrichtung in einem Fahrzeug (an einer Transportvorrichtung des Fahrzeugs) dienen.

Für letzteres verfügt die Vorrichtung über wenigstens ein Befestigungsmittel, beispielsweise über Gurte und Schnallen, am Grundelement, um das Grundelement an der bestehenden Krankentrage zu fixieren oder mit sonstigen fixen Gegenständen (wie Autositzen) bzw. mobilen Krankentragen in Krankenhäuser sicher zu verbinden. Damit kann die Vorrichtung derart in einem Fahrzeug befestigt werden, dass sie die sicherheitstechnischen Voraussetzungen, insbesondere hinsichtlich eines 10G-Crash-Test, erfüllt.

Ein weiterer Vorteil ist, dass sich diese Lösung nicht nur in der Mitte einer Krankentrage befestigen lässt, sondern flexibel im ganzen Bereich der Krankentrage montiert werden kann. Dadurch kann die Vorrichtung im Bereich einer Begleitperson in einem (Rettungs-)Fahrzeug positioniert werden, was der Begleitperson am "Rettersitz" im Fahrzeug ermöglich, ohne seinen Sicherheitsgurt zu lösen, an dem Neugeborenen zu arbeiten.

Zudem kann das Grundelement, nachdem der erste Innenraum evakuiert wurde, als eine Art stabiles Brett vorliegen und wie ein SpineBoard wirken und so das Lebewesen zu einem gewissen Grad immobilisieren.

Das Anlageelement kann vor dem Evakuieren an das Neugeborenen angeformt werden und stellt einen negativen Abdruck des Körpers des Neugeborenen dar. Nachdem das Anlageelement evakuiert wurde, fixiert dieses das Neugeborene in der Längs- sowie Querachse. In Verbindung mit dem Grundelement ergibt sich eine Ummantelung des Neugeborenen von zumindest drei Seiten - was eine Bewegung des Neugeborenen unterbindet.

Dadurch kann eine ausreichende medizinische Fixierung im Kopf und Halsbereich, bzw. der Seitenstabilität erreicht werden, welche selbst Neugeborene mit ihrer nicht ausgeprägten Wirbelsäule bzw. Nackenmuskulatur genügend stabilisiert bzw. immobilisiert. Eine Bewegung des Neugeborenen wird also so weit wie möglich verhindert.

Die Formgebung des Anlageelements ermöglicht aber auch einen nach oben offenen und zugänglichen Bereich zum Kopf sowie Brust des Neugeborenen. Dadurch kann das Neugeborenen bei Bedarf beatmen oder reanimiert werden, während es sich in der Vorrichtung befindet.

Die gesonderte Ausbildung und Evakuierbarkeit des ersten und zweiten Innenraums erlauben also einerseits eine bessere "Verhärtung" des Grundelements bzw. des Anlageelements beim evakuieren und andererseits ein besseres Anformen der genannten Elemente an einen Körper eines Lebewesens.

Im evakuierten Zustand verhindert eine solche Vorrichtung ob des Vakuums zunächst sogar eigenständig eine Unterkühlung bzw. den Abtransport von Wärme.

Die Vorrichtung bildet dabei eine besonders geformte Vakuummatratze. Die Innenräume der Vorrichtung werden also nach Art einer Vakuummatratze evakuiert.

Obige Ausführungen gelten analog für den Fall, dass das Grundelement aufgeblasen anstatt evakuiert wird. Dabei kann das Grundelement derart aufgeblasen werden, dass es sich verfestigt, wie diese beispielsweise auch bei aufblasbaren Stand-Up-Paddle-Boards der Fall ist.

Ein erfindungsgemäßes Verfahren umfasst folgende Schritte:
- Platzieren des Lebewesens auf dem Grundelement,
- Evakuieren oder Aufblasen des ersten Innenraums, wobei das Grundelement derart verfestigt wird, dass eine Wirbelsäule des Lebewesens zumindest teilweise immobilisiert wird,
- Anformen des Anlageelements an das Lebewesen, und
- Evakuieren des zweiten Innenraums, sodass das Lebewesen in der Vorrichtung gestützt und/oder stabilisiert ist.

Diese Schritte müssen nicht in der angegebenen Reihenfolge erfolgen, sondern können auch in einer anderen Reihenfolge oder gleichzeitig bzw. teilweise überschneidend durchgeführt werden.

Weitere bevorzugte Ausführungsformen sind in den abhängigen Ansprüchen definiert.

Vorteilhafterweise kann vorgesehen sein, dass das Grundelement einen Auflageabschnitt aufweist, auf welchem das Lebewesen zur Auflage bringbar ist.

Das Anlageelement kann mit dem Grundelement lösbar oder unlösbar verbunden sein.

Dadurch kann eine bessere Stabilisierung eines Lebewesens über die Vorrichtung erreicht werden.

Es kann vorgesehen sein, dass das wenigstens eine Anlageelement U-förmig ausgebildet ist.

Durch eine U-förmige Ausbildung des Anlageelements lässt sich dieses einfach und genau an einen Körper eines Lebewesens anformen. Insbesondere wenn es sich bei dem Lebewesen um ein Neugeborenes handelt, kann das Anlageelement so den Körper des Neugeborenen in seitlicher Richtung als auch im Bereich des Kopfs vollständig umschließen.

Bevorzugt kann vorgesehen sein, dass die Vorrichtung wenigstens ein erstes Ventil zur Evakuierung oder zum Aufblasen des Grundelements und wenigstens ein zweites Ventil zur Evakuierung des wenigstens einen Anlageelements aufweist.

Das stellt eine einfache Möglichkeit dar, den ersten Innenraum und den zweiten Innenraum gesondert voneinander zu evakuieren.

Es ist aber auch denkbar, dass die erste und der zweite Innenraum ein gemeinsames Ventil aufweisen, wobei das Ventil dazu ausgebildet ist, den ersten und den zweiten Innenraum gesondert voneinander zu evakuieren.

Es kann auch vorgesehen sein, dass in einem Zustand, in welchem ein Lebewesen in der Vorrichtung gestützt und/oder stabilisiert ist, zumindest ein Kopf und ein Brustbereich des in der Vorrichtung stabilisierten und/oder gestützten Lebewesens zugänglich ist.

Das ist besonders vorteilhaft, da dadurch das Lebewesen beispielsweise reanimiert und/oder beatmet werden kann, während es aber sicher in der Vorrichtung fixiert ist.

Bevorzugt kann vorgesehen sein, dass im ersten Innenraum und im zweiten Innenraum jeweils Granulat angeordnet ist.

Über ein Granulat kann die Anformung des Anlageelements an das Lebewesen und das Verhärten des Grundelements beim Evakuieren verbessert werden.

Gemäß einem weiteren Ausführungsbeispiel kann vorgesehen sein, dass im ersten Innenraum und/oder im zweiten Innenraum wenigstens ein Dämpfungselement, vorzugsweise umfassend ein Abstandsgewirke und/oder einen Schaumstoff, angeordnet ist.

Dadurch können Vibrationen in verschiedenen Frequenzbereichen, welche beispielsweise durch den Transport in einem Fahrzeug entstehen, eliminiert werden. Dadurch erfolgt eine stabilere und ruhigere Lagerung des Lebewesens. Ein solches Dämpfungselement kann auch als Polsterung für das Lebewesen in der Vorrichtung dienen.

Vorteilhafterweise kann das wenigstens eine Befestigungsmittel wenigstens einen Gurt und/oder wenigstens eine Schnalle umfassen.

Das stellt eine einfache Möglichkeit zur Umsetzung eines Befestigungsmittels dar. Zudem ist ein solches Befestigungsmittel universell einsetzbar, beispielsweise bei unterschiedlich ausgebildeten Tragen und/oder Sitzen.

Es sind aber auch andere Ausbildungen eines Befestigungsmittels denkbar.

Es kann vorgesehen ein, dass an der Vorrichtung, vorzugsweise an dem Grundelement, von dem wenigstens einen Befestigungsmittel gesonderte Haltegriffe und/oder Haltegurte angeordnet sind.

Das macht es, beispielsweise für Rettungskräfte, leichter, eine Vorrichtung zu transportieren und handzuhaben.

Gemäß einem weiteren Ausführungsbeispiel kann das Grundelement rechteckförmig ausgebildet sein.

Ein rechteckförmiges Grundelement lässt sich einfach herstellen, ist einfach handzuhaben und ist mit einer Vielzahl and Trage und/oder Sitzen kompatibel.

Selbstverständlich sind jedoch auch andere Formen für das Grundelement denkbar.

Es kann weiters vorgesehen sein, dass das wenigstens eine Anlageelement zumindest ein Sicherungsmittel, vorzugsweise einen Gurt, umfasst, um das Lebewesen zusätzlich im Anlageelement zu fixieren.

Mittels des zumindest einen Sicherungsmittels kann das Anlageelement geschlossen bzw. fixiert werden. Dadurch kann eine zusätzliche Fixierung des Lebewesens in der Vorrichtung ermöglicht werden. Im Falle eines falschen oder fehlerhaften Anformens des Anlageabschnitts an das Lebwesens ist dies besonders vorteilhaft.

Die Zugänglichkeit zum Lebewesen von oben kann bei einem solchen Sicherungsmittel aber weiterhin gegeben sein.

Besonders bevorzugt kann vorgesehen sein, dass die Vorrichtung wenigstens eine Temperiervorrichtung zum Temperieren eines Lebewesens, insbesondere zum Kühlen und/oder Wärmen, umfasst.

Eine solche Temperiervorrichtung ermöglicht es, ein Lebewesen in der Vorrichtung zu temperieren. Das Lebewesen kann also beispielsweise gewärmt werden, um ein Unterkühlen zu verhindern. Es ist beispielsweise auch möglich das Lebewesen zu kühlen, um Schäden beispielsweise am Gehirn zu vermeiden.

Dahingehend kann vorgesehen sein, dass die wenigstens eine Temperiervorrichtung im ersten Innenraum und/oder im zweiten Innenraum angeordnet ist.

Durch ein solche direkte Integration in die Vorrichtung ist keine gesonderte Temperiervorrichtung notwendig, wodurch die Vorrichtung einfacher handzuhaben ist.

Besonders bevorzugt kann vorgesehen sein, dass die Temperiervorrichtung zum Temperieren eines Lebewesens, insbesondere eines Menschen,
- eine Hülle mit einer ersten Seite zur Auflage eines Lebewesens, und
- wenigstens ein Temperierelement, welches innerhalb der Hülle angeordnet ist, wobei mittels des wenigstens einen Temperierelements ein Wärmestrom erzeugbar ist,
umfasst, wobei die Temperiervorrichtung wenigstens ein innerhalb der Hülle angeordnetes Wärmeübertragungselement umfasst, mittels welchem der von dem wenigstens einen Temperierelement erzeugte Wärmestrom auf die erste Seite der Hülle übertragbar ist, um ein auf der Hülle befindliches Lebewesen zu wärmen und/oder zu kühlen.

Dadurch ist es möglich, über das Temperierelement einen Wärmestrom bereitzustellen, welcher eine wärmende und/oder auch eine kühlende Wirkung auf die erste Seite der Hülle hat. Demnach kann ein auf der Hülle befindliches Lebewesen gewärmt und/oder gekühlt werden.

Ein Wärmeübertragungselement hat den Vorteil, dass kein großflächiges Temperierelement vorgesehen werden muss. Damit können kleine Temperierelemente vorgesehen werden, welche kostengünstig und wenig anfällig für Beschädigungen sind. Auch eine zusätzliche Stabilisierungsschicht kann eingespart werden.

Betreffend ein kühlend wirkendes Temperierelement ermöglicht ein Wärmeübertragungselement überhaupt erst eine sinnvolle Umsetzung einer Temperiervorrichtung mir ausreichender Kühlleistung. Flächige Temperierelemente, welche eine kühlende Wirkung erzeugen können und für den Einsatz in gattungsgemäßen Temperiervorrichtungen geeignet sind, sind nämlich nicht bekannt oder zu teuer.

Hinsichtlich eines Verfahrens kann vorgesehen sein, dass die Vorrichtung in einem weiteren Schritt über das wenigstens eine Befestigungsmittel an einer Transportvorrichtung, vorzugsweise einer Trage oder einem Sitz, befestigt wird.

Weitere Einzelheiten und Vorteile bevorzugter Ausführungsformen der Erfindung werden anhand der Figurenbeschreibung unter Bezugnahme auf die Zeichnungen im Folgenden näher erläutert. Darin zeigen:
- Fig. 1A: eine schematische Draufsicht einer Vorrichtung,
- Fig. 1B: eine schematische Unteransicht der Vorrichtung,
- Fig. 2: eine schematische Draufsicht der Vorrichtung mit einem darin fixierten Lebewesen,
- Fig. 3: eine schematische Draufsicht der Vorrichtung auf einem Transportmittel,
- Fig. 4A: eine schematische Schnittdarstellung eines ersten Ausführungsbeispiels der Vorrichtung,
- Fig. 4B: eine schematische Schnittdarstellung eines zweiten Ausführungsbeispiels der Vorrichtung, und
- Fig. 4C: eine schematische Schnittdarstellung eines dritten Ausführungsbeispiels der Vorrichtung.

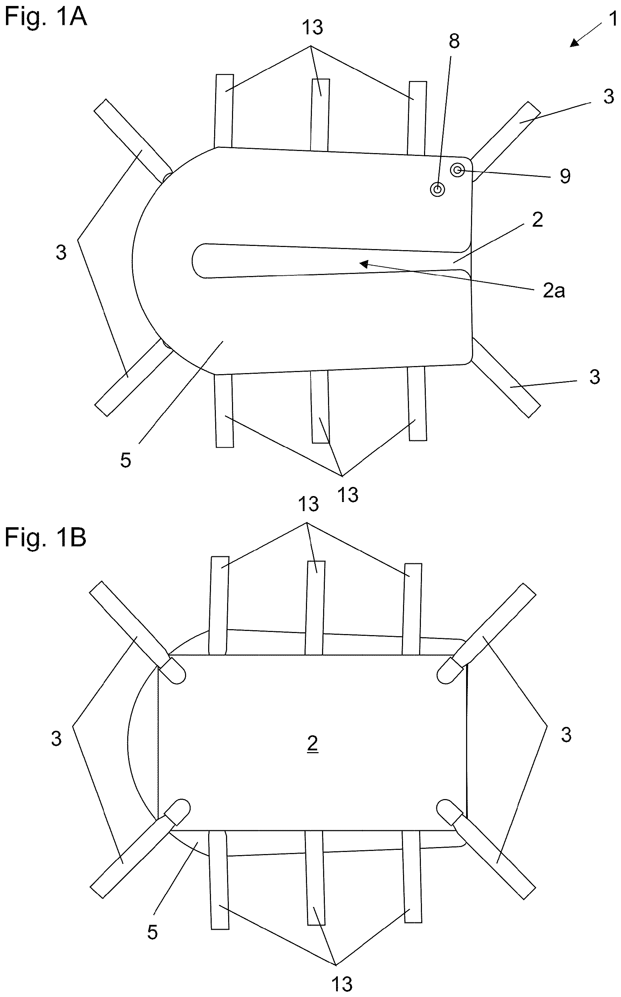

Die Fig. 1A zeigt eine schematische Draufsicht einer Vorrichtung 1 und die Fig. 1B eine schematische Unteransicht der Vorrichtung 1.

Es ist erkennbar, dass die Vorrichtung 1 ein im Wesentlichen rechteckförmiges Grundelement 2 aufweist, an welchem Befestigungsmittel 3 angeordnet sind. Im vorliegenden Ausführungsbeispiel sind die Befestigungsmittel 3 als Gurte ausgebildet. Es sind jedoch auch andere Arten von Befestigungsmittel 3 denkbar.

Auf dem Grundelement 2 ist ein mit dem Grundelement 2 verbundenes Anlageelement 5 angeordnet. Das Anlageelement 5 ist annähernd U-förmig ausgebildet.

Sowohl das Grundelement 2 als auch das Anlageelement 3 sind in diesem Ausführungsbeispiel elastisch ausgebildet. Das Anlageelement 3 ist an den Körper eines Lebewesens anformbar.

Am Grundelement 2 ist ein Auflageabschnitt 2a ausgebildet, auf welchem ein Lebewesen zur Auflage kommen kann. Der Auflageabschnitt 2a kann sich dabei auch teilweise unterhalb des Anlageabschnitts 5 erstrecken.

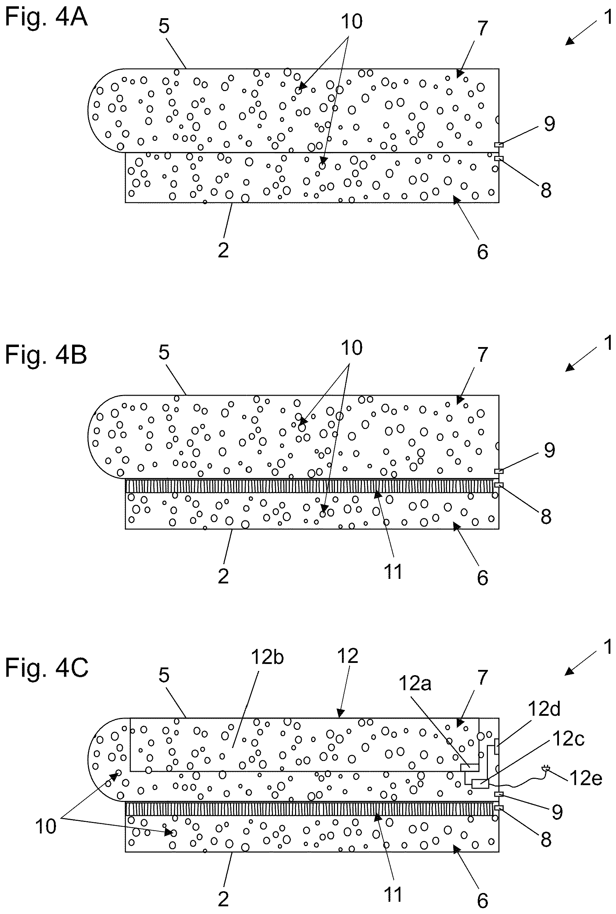

Zudem sind ein erstes Ventil 8 zur Evakuierung oder zum Aufblasen des Grundelements 2 und ein zweites Ventil 9 zur Evakuierung des Anlageelements 5 erkennbar. Im vorliegenden Ausführungsbeispiel kann das erste Ventil 8 durch das Anlageelement 5 hindurch zum Grundelement 2 geführt sein. Grundsätzlich können das erste Ventil 8 und das zweite Ventil 9 an beliebigen Stellen der Vorrichtung 1 platziert werden. Eine alternative Platzierung ist beispielsweise in den Fig. 4A bis 4C erkennbar.

Am Anlageelement 5 sind weiters mehrere Sicherungsmittel 13 mit jeweils einem Gurt 13a und einer Schnalle 13b erkennbar. Über diese Sicherungsmittel 13 können die beiden Schenkel des U-förmigen Anlageabschnitts 5 zusammengehalten werden. In anderen Worten kann das Anlageelement 5 geschlossen bzw. fixiert werden. Dadurch kann eine zusätzliche Fixierung des Lebewesens in der Vorrichtung ermöglicht werden.

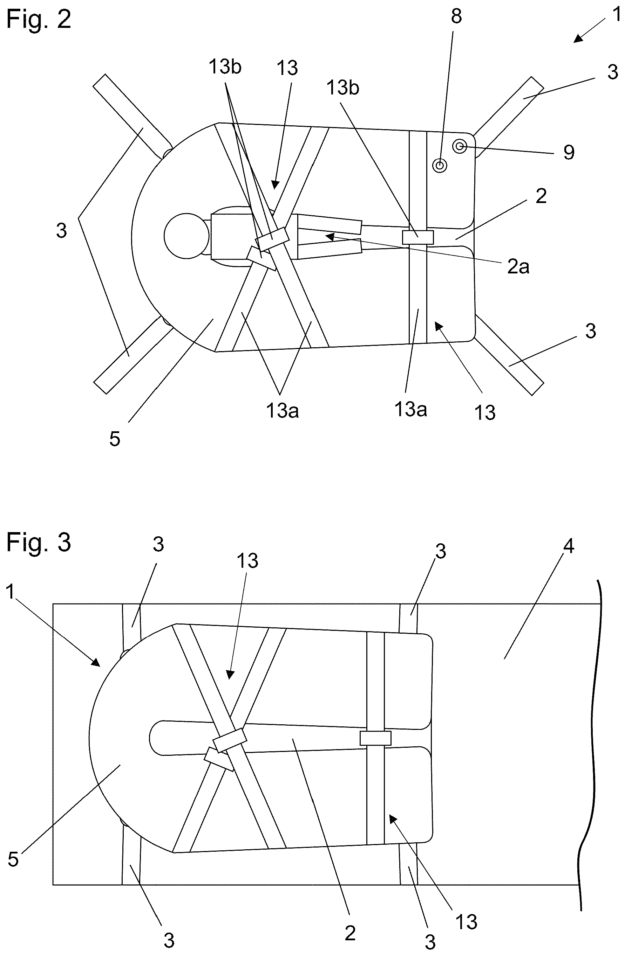

Das ist in der Fig. 2, welche eine schematische Draufsicht der Vorrichtung 1 mit einem darin fixierten Lebewesen zeigt, gut ersichtlich.

Das Lebewesen ist in der Fig. 2 ein Neugeborenes, was an der kleinen Körpergröße erkennbar ist.

Der Anlageabschnitt 5 ist um das Lebewesen geformt und evakuiert. Auch das Grundelement 2 ist evakuiert. Das Grundelement 2 ist demnach verhärtet und bildet eine Art "hartes Brett" aus. Das Grundelement 32 fungiert somit als SpineBoard für das Lebewesen und immobilisiert insbesondere die Wirbelsäule des Lebewesens.

Es ist auch denkbar, dass das Grundelement 2 aufgeblasen wird, um es zu verhärten.

Über den angeformten Anlageabschnitt ist das Lebewesen sicher in der Vorrichtung 5 fixiert. Zudem sind auch die Sicherungsmittel 13 angelegt, welche eine zusätzliche Fixierung des Lebewesens ermöglichen.

Die Zugänglichkeit zum Lebewesen im Bereich des Kopfs und der Brust von oben ist vollständig gegeben, um das Lebewesen gegebenenfalls beatmen und/oder reanimieren zu können.

Die Fig. 3 zeigt eine schematische Draufsicht der Vorrichtung lauf einem Transportmittel 4. Das Transportmittel 4 ist hier als Trage ausgebildet.

Die Vorrichtung 1 ist über die Befestigungsmittel 3 mit der Transportvorrichtung 4 verbunden. Diese Verbindung ist dergestalt, dass alle sicherheitstechnischen Anforderungen erfüllt werden. Insbesondere kann auch ein 10G-Crash-Test bestanden werden.

Die Transportvorrichtung 4 kann aber beispielsweise auch ein (Fahrzeug-)Sitz, ein Krankenbett oder dergleichen sein.

Die Fig. 4A zeigt eine schematische Schnittdarstellung eines ersten Ausführungsbeispiels der Vorrichtung 1.

Es ist erkennbar, dass das Grundelement einen ersten Innenraum 6 und das Anlageelement 5 einen zweiten Innenraum 7 aufweist. Der erste Innenraum 6 und der zweite Innenraum 7 sind dabei gesondert voneinander ausgebildet und auch gesondert voneinander evakuierbar. Für letzteres sind ein mit dem ersten Innenraum 6 verbundenes erstes Ventil 8 und ein mit dem zweiten Innenraum 7 verbundenes zweites Ventil 9 vorgesehen.

Sowohl im ersten Innenraum 6 als auch im zweiten Innenraum 7 ist Granulat 10 angeordnet. Im nicht-evakuierten Zustand kann das Anlageelement 5 an das Lebewesen angeformt werden, wobei sich das Granulat 10 im zweiten Innenraum 7 entsprechend verformt. Nach dem Evakuieren des zweiten Innenraums 7 ist das Anlageelement 5 ob des Granulats 10 in seiner angeformeten Form verfestigt und fixiert so das Lebewesen.

Im Grundelement 2 kann über das Granulat ein Verhärten des Grundelements 2 erreicht werden.

Die Fig. 4B zeigt eine schematische Schnittdarstellung eines zweiten Ausführungsbeispiels der Vorrichtung 1. Diese unterscheidet sich vom ersten Ausführungsbeispiel lediglich dadurch, dass im ersten Innenraum 6 ein Dämpfungselement 11 in Form eines Abstandsgewirkes angeordnet ist. Über das Dämpfungselement 11 können Vibrationen in der Vorrichtung reduziert und/oder eliminiert werden.

Das Dämpfungselement 11 kann auch nur im zweiten Innenraum 7 oder in beiden Innenräumen 6, 7 vorgesehen sein.

Die Fig. 4C zeigt eine schematische Schnittdarstellung eines dritten Ausführungsbeispiels der Vorrichtung 1. Dieses unterscheidet sich vom zweiten Ausführungsbeispiel dadurch, dass im zweiten Innenraum 7 eine Temperiervorrichtung 12 vorgesehen ist.

Die Temperiervorrichtung 12 umfasst dabei ein Temperierelement 12a zum Erzeugen eines Wärmestroms. Ein Wärmestrom ist dabei im physikalischen Sinne als die Menge an Wärme, welche von einem Ort hoher Temperatur zu einem Ort niedriger Temperatur übertragen wird, zu verstehen. Je nach Richtung des Wärmestroms kann der durch das Temperierelement erzeugte Wärmestrom demnach eine Erwärmung oder eine Abkühlung zur Folge haben.

Eine Hülle der Temperiervorrichtung 12 wird hier durch eine Wandung des Anlageelements 5 bzw. des zweiten Innenraums 7 ausgebildet.

Die Temperiervorrichtung 12 umfasst weiters ein Wärmeübertragungselement 12b mittels welchem der von dem wenigstens einen Temperierelement 12a erzeugte Wärmestrom auf die erste Seite der Hülle übertragbar ist, um ein auf der Hülle befindliches Lebewesen zu wärmen und/oder zu kühlen

Somit kann über die Temperiervorrichtung 12 ein in der Vorrichtung fixiertes Lebewesen temperiert werden.

Das Temperierelement 12a kann beispielsweise ein Peltier-Element umfassen.

Das Wärmeübertragungselement 12b kann eine wärmeleitfähige Folie umfassen.

Die Temperiervorrichtung 12 umfasst darüber hinaus eine Regelungsvorrichtung 12c zur Regelung und/oder Steuerung des Temperierelements 12a. Zudem ist auch ein Betätigungselement 12d zum Bedienen der Temperiervorrichtung 12 vorgesehen. Über eine Schnittstelle 12e in Form eines Steckers kann die Temperiervorrichtung 12 mit Energie versorgt werden.

Es ist auch denkbar, dass ein Temperatursensor vorgesehen ist, wobei die Temperiervorrichtung 12 in Abhängigkeit der Messwerte des Temperatursensor regelbar ist.

Die Temperiervorrichtung 12 kann auch nur erste Innenraum 6 oder in beiden Innenräumen 6, 7 vorgesehen sein.

### Bezugszeichenliste:

- 1: Vorrichtung
- 2: Grundelement
2a Auflageabschnitt
- 3: Befestigungsmittel
- 4: Transportvorrichtung
- 5: Anlageelement
- 6: Erster Innenraum
- 7: Zweiter Innenraum
- 8: Erstes Ventil
- 9: Zweites Ventil
- 10: Granulat
- 11: Dämpfungselement
- 12: Temperiervorrichtung
12a Temperierelement
12b Wärmeübertragungselement
12c Regelungsvorrichtung
12d Betätigungselement
12e Schnittstelle
- 13: Sicherungsmittel
13a Gurt
13b Schnalle

## Patentansprüche

1. Vorrichtung (1) zur Fixierung eines Lebewesens, insbesondere eines Neugeborenen, umfassend
- ein Grundelement (2) mit wenigstens einem Befestigungsmittel (3) zur Befestigung der Vorrichtung (1) an einer Transportvorrichtung (4), vorzugsweise einer Trage oder einem Sitz, und
- wenigstens ein am Grundelement (2) angeordnetes, flexibles Anlageelemente (5), wobei das Anlageelement (5) am Lebewesen festlegbar ist,
wobei das Grundelement (2) wenigstens einen ersten evakuierbaren oder aufblasbaren Innenraum (6) und das wenigstens eine Anlageelement (5) wenigstens einen zweiten evakuierbaren Innenraum (7) aufweist, **dadurch gekennzeichnet, dass** der wenigstens eine erste Innenraum (6) und der wenigstens eine zweite Innenraum (7) gesondert voneinander ausgebildet und/oder gesondert voneinander evakuierbar sind.

2. Vorrichtung (1) nach Anspruch 1, wobei das Grundelement (2) einen Auflageabschnitt (2a) aufweist, auf welchem das Lebewesen zur Auflage bringbar ist.

3. Vorrichtung (1) nach einem der Ansprüche 1 oder 2, wobei das wenigstens eine Anlageelement (5) U-förmig ausgebildet ist.

4. Vorrichtung (1) nach einem der Ansprüche 1 bis 3, wobei die Vorrichtung (1) wenigstens ein erstes Ventil (8) zur Evakuierung oder zum Aufblasen des Grundelements (2) und wenigstens ein zweites Ventil (9) zur Evakuierung des wenigstens einen Anlageelements (5) aufweist.

5. Vorrichtung (1) nach einem der Ansprüche 1 bis 4, wobei in einem Zustand, in welchem ein Lebewesen in der Vorrichtung (1) fixiert ist, zumindest ein Kopf und ein Brustbereich des in der Vorrichtung fixierten Lebewesens zugänglich ist.

6. Vorrichtung (1) nach einem der Ansprüche 1 bis 5, wobei im ersten Innenraum (6) und im zweiten Innenraum (7) jeweils Granulat (10) angeordnet ist.

7. Vorrichtung (1) nach einem der Ansprüche 1 bis 6, wobei im ersten Innenraum (6) und/oder im zweiten Innenraum (7) wenigstens ein Dämpfungselement (11), vorzugsweise umfassend ein Abstandsgewirke und/oder einen Schaumstoff, angeordnet ist.

8. Vorrichtung (1) nach einem der Ansprüche 1 bis 7, wobei das wenigstens eine Befestigungsmittel (3) wenigstens einen Gurt und/oder wenigstens eine Schnalle umfasst.

9. Vorrichtung (1) nach einem der Ansprüche 1 bis 8, wobei an der Vorrichtung (1), vorzugsweise an dem Grundelement (2), von dem wenigstens einen Befestigungsmittel (3) gesonderte Haltegriffe und/oder Haltegurte angeordnet sind.

10. Vorrichtung (1) nach einem der Ansprüche 1 bis 9, wobei das Grundelement (2) rechteckförmig ausgebildet ist.

11. Vorrichtung (1) nach einem der Ansprüche 1 bis 10, wobei die Vorrichtung (1) wenigstens eine Temperiervorrichtung (12) zum Temperieren eines Lebewesens, insbesondere zum Kühlen und/oder Wärmen, umfasst.

12. Vorrichtung (1) nach Anspruch 11, wobei die wenigstens eine Temperiervorrichtung (12) im ersten Innenraum (6) und/oder im zweiten Innenraum (7) angeordnet ist.

13. Vorrichtung (1) nach einem der Ansprüche 11 oder 12, wobei die Temperiervorrichtung (12) zum Temperieren eines Lebewesens, insbesondere eines Menschen,
- eine Hülle mit einer ersten Seite zur Auflage eines Lebewesens, und
- wenigstens ein Temperierelement (12a), welches innerhalb der Hülle angeordnet ist, wobei mittels des wenigstens einen Temperierelements (12a) ein Wärmestrom erzeugbar ist,
umfasst, wobei die Temperiervorrichtung (12) wenigstens ein innerhalb der Hülle angeordnetes Wärmeübertragungselement (12b) umfasst, mittels welchem der von dem wenigstens einen Temperierelement (12a) erzeugte Wärmestrom auf die erste Seite der Hülle übertragbar ist, um ein auf der Hülle befindliches Lebewesen zu wärmen und/oder zu kühlen.

14. Verfahren zum Fixieren eines Lebewesens, insbesondere eines Neugeborenen, mit einer Vorrichtung (1) nach einem der Ansprüche 1 bis 13, umfassend folgende Schritte:
- Platzieren des Lebewesens auf dem Grundelement (2),
- Evakuieren oder Aufblasen des ersten Innenraums (6), wobei das Grundelement (2) derart verfestigt wird, dass eine Wirbelsäule des Lebewesens zumindest teilweise immobilisiert wird,
- Anformen des Anlageelements (5) an das Lebewesen, und
- Evakuieren des zweiten Innenraums (7), sodass das Lebewesen in der Vorrichtung (1) gestützt und/oder stabilisiert ist.

15. Verfahren nach Anspruch 14, wobei die Vorrichtung (1) in einem weiteren Schritt über das wenigstens eine Befestigungsmittel an einer Transportvorrichtung (4), vorzugsweise einer Trage oder einem Sitz, befestigt wird.
